# EUROPÄISCHE PATENTSCHRIFT

(11) **EP 1 596 703 B1**
(45) Veröffentlichungstag und Bekanntmachung des Hinweises auf die Patenterteilung: **08.04.2009**
(21) Anmeldenummer: 04706138.7
(22) Anmeldetag: 29.01.2004
(51) Int. Cl.: A61B 1/00

(54) **VERFAHREN ZUM MONTIEREN EINES ENDOSKOPES**
METHOD FOR ASSEMBLING AN ENDOSCOPE
PROCEDE POUR MONTER UN ENDOSCOPE

(30) Priorität: 18.02.2003 DE 10307903
(43) Veröffentlichungstag der Anmeldung: 23.11.2005
(73) Patentinhaber: Karl Storz GmbH & Co. KG, 78532 Tuttlingen (DE)
(72) Erfinder: RENNER, Klaus, 78576 Liptingen (DE); BRÜSEHABER, Steffen, 78532 Tuttlingen (DE)
(74) Vertreter: Weller, Wolfgang
(86) Internationale Anmeldenummer: PCT/EP2004/000766
(87) Internationale Veröffentlichungsnummer: WO 2004/073507

(56) Entgegenhaltungen:
- EP-A- 0 459 415
- EP-A- 1 271 213
- DE-A- 3 738 451
- DE-A- 3 822 885
- DE-A- 4 207 092
- GB-A- 1 463 350
- US-A- 5 662 817
- PATENT ABSTRACTS OF JAPAN Bd. 1997, Nr. 09, 30. September 1997 (1997-09-30) & JP 9 127379 A (FURUKAWA ELECTRIC CO LTD:THE), 16. Mai 1997 (1997-05-16)

## Beschreibung

Die Erfindung betrifft ein Verfahren zum Montieren eines Endoskopes mit einem ersten rohrförmigen Kanal zur Aufnahme von bildleitenden Bauelementen und einem zweiten Kanal zur Aufnahme von Lichtleitern.

Die Erfindung betrifft gleichermaßen ein derartiges Endoskop.

Endoskope haben im medizinischen Bereich eine weite Verbreitung gefunden und öffneten den Weg zu der sogenannten "minimalinvasiven Chirurgie". Zwischenzeitlich hat sich ein weiteres Einsatzgebiet der sogenannten "industriellen Endoskopie" aufgetan, bei der die Endoskope zur visuellen Beobachtung von Hohlräumen, beispielsweise in Motoren, Karosserien, Flugzeugturbinen, Gebäuden etc. herangezogen werden.

Derartige Endoskope weisen einen ersten rohrförmigen Kanal zur Aufnahme von bildleitenden Elementen auf. Diese bildleitenden Elemente sind Elemente eines optischen Systems, wie Linsen, insbesondere Stablinsen, Abstandhalter, Blenden, Prismen, Filter oder dergleichen. Diese leiten das Bild von distal nach proximal. Bei einer elektronischen Bildverarbeitung ist eine Miniaturkamera in Form eines CCD-Chips vorhanden, der die optischen Signale in elektrische Signale umwandelt.

Für das notwendige Beobachtungslicht dienen Lichtleiter, die von einer Lichtquelle das Licht von proximal nach distal leiten. Diese Lichtleiter werden üblicherweise aus einem Bündel an Glasfasern gebildet.

Bei der Montage des Endoskopes werden die Lichtleiter in Form eines Bündels von Glasfasern direkt in einen zweiten Kanal eingebracht, und dieser wird dann beidseits verschlossen. Dieser zweite Kanal wird gebildet, indem ein zweites Rohr über den ersten rohrförmigen Kanal geschoben wird und diesen dann umgibt, so dass ein Spalt zwischen dem ersten rohrförmigen Kanal und dem zweiten Rohr den zweiten Kanal als Kanal mit dem Querschnitt eines durchgehenden Rings oder mit einem sichelförmigen ggf. nicht durchgehenden Querschnitt bildet. Derartige Endoskope weisen einen langerstreckten Schaft auf, in dem die beiden Kanäle zur Aufnahme der bildleitenden und lichtleitenden Bauelemente vorgesehen sind. Proximalseitig mündet der Schaft in ein Endoskopgehäuse, dessen proximales Ende wiederum, je nach Ausgestaltung, eine Okularmuschel oder einen Anschluss an ein Kameramodul aufweist. Auf Grund der Biegsamkeit der dünnen Lichtleiter aus Glasfasern ist es möglich, diese zu einem quer zur Längsachse des Schaftes abstehenden Lichtleiterstutzen zu führen. In einem endfertig montierten Endoskop erstreckt sich im zweiten Kanal ein erster langerstreckter Abschnitt des Lichtleiters längs der Schaftachse, der dann im Gehäuse etwa um 90° seitlich abgebogen über eine entsprechende Biegung oder Krümmung zum Lichtleiterstutzen bzw. -anschluss führt.

Üblicherweise ist der Schaft eines solchen Endoskopes aus einem Außenrohr ausgebildet, in dem ein Innenrohr aufgenommen ist, in dessen Innenraum der erste Kanal ausgebildet ist und der zur Aufnahme der bildleitenden Elemente dient. Ist das Innenrohr koaxial zum Außenrohr angeordnet, ist zwischen der Außenseite des Innenrohrs und der Innenseite des Außenrohrs ein hohlzylindrischer Raum gebildet, der den zweiten Kanal zur Aufnahme der Lichtleiter im Schaftbereich darstellt.

Ist das Innenrohr seitlich versetzt im Außenrohr angeordnet, meist so, dass es längs einer äußeren Mantellinie an einer inneren Mantellinie der Innenseite des Außenrohrs anliegt, so entsteht ein mondsichelförmiger Raum, der den zweiten Kanal zur Aufnahme der Lichtleiter darstellt.

Es wurde festgestellt, dass die Montage des Lichtleiters in Form eines losen Bündels an Glasfasern eine gewisse Übung und ein erhebliches Geschick erfordert. Dennoch konnte nicht ausgeschlossen werden, dass einige Glasfasern bei der Montage brechen und nicht zum Lichtleiten herangezogen werden können. Ferner wurde festgestellt, dass nach zahlreichen Sterilisierzyklen Feuchtigkeit oder sonstige Kontaminationen in den zweiten Kanal eindringen können und die Lichtleitqualität nachteilig beeinflussen.

Die distal- und proximalseitige Fixierung des Glasfaserbündels erfolgt über Verklebungen. Dabei wurde ein Kriechen des Klebemittels von den Fixierstellen weg längs der Glasfasern festgestellt, was zu unerwünschten Verklebungen von einzelnen Glasfasern untereinander und von Glasfasern am Innen- oder Außenrohr abseits der endseitigen Fixierstellen führt. Dies führt bei thermischen und mechanischen Dehnungsspannungen und Schocks zum Bruch von Glasfasern, die dann nicht mehr zur Lichtleitung dienen können.

Aus der DE 37 38 451 A1 ist ein Endoskop bekannt, das einen ersten rohrförmigen Kanal zur Aufnahme von bildleitenden Bauelementen und einen zweiten Kanal zur Aufnahme von Lichtleitern aufweist, wobei die Lichtleiter in einem flexiblen Schlauch eingebracht werden.

Aus der DE 38 22 885 A1 ist ein optisches Faserbündel für ein Endoskop beschrieben, bei dem eine Anzahl von optischen Fasern an beiden Enden miteinander verbunden sind. Die Lichtleiter sind in einem Schlauch mittels Anziehen eines Gewindes fixiert.

Aus der US 5,662,817 A ist ein Schlauch bekannt, in dem Lichtleiter aufgenommen sind, die beidseits des Schlauches vorragen.

Aus der JP 56159607 A sind Lichtleiter für ein Endoskop beschrieben, die in einem Schlauch fixiert werden. Dabei enden die Lichtleiter auf Höhe der Schlauchenden und können dort über ein Klebemittel fixiert werden.

Aus der JP 61259204 sind Lichtleiter für ein Endoskop beschrieben, die in einem Schlauch fixiert sind. Auch hier können die Lichtleiter mit einem Klebemittel fixiert werden.

Aus der US 6,323,742 B1 ist ein Lichtleiter offenbart, der in einem Schlauch durch Verklemmen fixiert wird.

Es ist Aufgabe der vorliegenden Erfindung ein Verfahren zum Montieren eines Endoskops zu schaffen, das einfach durchzuführen ist und das eine auf Dauer hervorragende Lichtleistung gewährleistet und das zu einem Endoskop führt, das diese vorgenannten Eigenschaften aufweist.

Erfindungsgemäß wird die Aufgabe durch ein Verfahren zum Montieren eines Endoskopes mit einem ersten rohrförmigen Kanal zur Aufnahme von bildleitenden

Bauelementen und mit einem zweiten Kanal zur Aufnahme von Lichtleitern gelöst, wobei die Lichtleiter in einem flexiblen Schlauch eingebracht werden und der Zusammenbau aus Schlauch und Lichtleiter in dem zweiten Kanal eingebracht wird und die Lichtleiter im Schlauch fixiert werden, wobei die Lichtleiter mit einer solchen Länge versehen werden, dass sie beidseits des Schlauches hervorragen und wobei ein Schlauch eingesetzt wird, der aus schrumpffähigem Material besteht und der Schlauch nach dem Einbringen in den zweiten Kanal geschrumpft wird.

Die Aufgabe wird bei einem Endoskop mit einem ersten rohrförmigen Kanal zur Aufnahme von bildleitenden Bauelementen mit einem zweiten Kanal zur Aufnahme von Lichtleitern dadurch gelöst, dass die Lichtleiter in einem geschrumpften flexiblen Schlauch aufgenommen sind und der Zusammenbau aus Lichtleiter und Schlauch im zweiten Kanal aufgenommen ist, wobei die Lichtleiter im Schlauch fixiert sind und eine solche Länge aufweisen, dass diese beidseits des Schlauches hervorragen.

Durch den Vormontageschritt ist es nun möglich, die Lichtleiter, ggf. unter Zuhilfenahme von Montagehilfen, in einen flexiblen Schlauch einzubringen. Dieses Einführen oder

Einbringen in den flexiblen Schlauch ist ohne Brechen von einzelnen Lichtleitern möglich. Dieser Zusammenbau wird anschließend in den zweiten Kanal des Endoskopes eingebracht, was wesentlich einfacher durchzuführen ist als dies mit einem losen Lichtleiterbündel der Fall wäre. Der flexible Schlauch bewirkt auch die Krümmung oder Biegung des im Schlauch aufgenommenen Lichtleiterbündels, um den Zusammenbau zu dem seitlich abgewinkelten Lichtleiteranschluss zu bringen oder in diesen einzufädeln. Durch Manipulationen an dem äußeren, die inneren Glasfasern umhüllenden Schlauch können dann Sitz- oder Lagekorrekturen durch Hin- und Herschieben durchgeführt werden, wobei bei diesen Manipulationen nicht das Risiko besteht, dass einzelne Glasfasern sich verdrillen, verwinden oder gar abbrechen und diese dann nicht mehr zur Lichtleitung herangezogen werden können.

Der flexible Schlauch schützt außerdem die Lichtleitfasern im späteren Einsatz des Endoskopes vor radialem Eindringen von Feuchtigkeit oder Kontaminationen, so dass nicht nur die Montage vereinfacht wird, sondern auch zur Betriebssicherheit und erhöhter Lebensdauer der lichtleitenden Elemente beigetragen wird. Insbesondere in dem Krümmungsbereich, also dem Bereich, in dem die Leiter proximalseitig aus der Langerstreckung des Schaftes zum seitlichen Lichtleiteranschluss geführt sind, sind diese Lichtleiter nunmehr besonders geschützt, so dass keine Beeinträchtigungen durch andere Bauteile im Gehäuse des Endoskopes entstehen können. Selbst wenn sich innerhalb des Endoskopes auf Grund von Erschütterungen oder dergleichen irgendwelche Teile ablösen oder bewegen, können diese nicht unmittelbar auf das Lichtleiterbündel treffen, da dieses in dem kritischen Bereich durch den es umgebenden flexiblen Schlauch geschützt ist. Bei Revisionen oder allfälligen Reparaturen können dementsprechend auch Handhabungen mit Werkzeugen im Bereich des Gehäuses durchgeführt werden, ohne dass die Gefahr besteht, dass man auf freiliegende Bereiche der empfindlichen Lichtleiter in Form von Glasfasern trifft.

Die Lichtleiter weisen eine solche Länge auf, dass sie beidseits des Schlauches hervorragen.

Diese Maßnahme hat den Vorteil, dass die beidseits hervorragenden Enden dazu herangezogen werden, um am jeweiligen proximalen bzw. distalen Ende wie üblich fixiert zu werden, beispielsweise durch entsprechende Verklebungen. Diese Verklebungen werden so bewerkstelligt, dass die Enden der Lichtleiter lagefixiert werden und zugleich das Klebemittel einen dichten Abschluss am proximalen Ende bzw. distalen Ende des Endoskopes bzw. des lichtleitenden Pfades bildet.

Diese Maßnahme hat den Vorteil, dass das Bündel an Lichtleitern an diesen endseitigen Stellen lagefest und dichtend fixiert ist, dennoch aber zwischen diesen Bereichen axiale Dehnungen bei den Sterilisationszyklen möglich sind, die bei erhöhten Temperaturen (bis zu ca. 170 °C), in der Regel durch Autoklavieren, durchgeführt werden. Der die Lichtleitfasern zwischen den endseitigen Fixierstellen umgebende Schlauch schützt diese empfindlichen Lichtleitfasern, erlaubt aber dennoch die Dehnbewegungen bei schwankenden Temperaturen auf der gesamten Dehnstrecke zwischen dem proximalen und dem distalen Endbereich. Es wird ein Schlauch eingesetzt, der aus schrumpffähigem Material besteht.

Diese Maßnahme hat den Vorteil, dass nach Einbringen der Lichtleiter das Material des Schlauches geschrumpft werden kann, so dass die Lichtleiter zusätzlich fixiert sind.

Dabei wird der Schlauch nach dem Einbringen in den zweiten Kanal geschrumpft.

Diese Maßnahme hat beispielsweise bei dem zuvor erwähnten sichelförmigen zweiten Kanal den Vorteil, dass der Schlauch zunächst als loses lockeres Gebilde in eine solche Form eingeschoben, dieser angepasst wird und nachher durch den Schrumpfvorgang unter Anpassung an diese Geometrie fixiert wird.

Die Lichtleiter werden im Schlauch fixiert.

Diese Maßnahme hat den Vorteil, dass die Lichtleiter im Bereich des Schlauches lagefixiert sind, dennoch aber auf Grund der Flexibilität Dehnungsbewegungen zum Spannungsausgleich möglich sind.

In einer weiteren Ausgestaltung der Erfindung sind die Lichtleiter im Bereich der Schlauchenden fixiert.

Diese Maßnahme hat den Vorteil, dass durch die Fixierung eine definierte Lage der Lichtleiter im Bereich der Enden des Schlauches gewährleistet ist, im Bereich zwischen diesen Enden aber ausreichend Bewegungsfreiheit gegeben ist, um Dehnungen bei Temperaturschwankungen oder Biegungen oder Krümmungen zum Führen der Lichtleiter zu ermöglichen. Die Fixierung verhindert auch, dass beim Handhaben während der Montage versehentlich einige Lichtleitfaserenden in den Schlauch eingezogen werden und sich dort verheddern und verkanten. Deren Enden reichen dann nicht mehr bis an die distale bzw. proximale Endfläche und somit können diese Lichtleiter nicht mehr zur Lichtleitung herangezogen werden.

In einer weiteren Ausgestaltung der Erfindung sind die Lichtleiter über ein Klebemittel im Schlauch fixiert.

Diese Maßnahme hat den Vorteil, dass im Bedarfsfall die Lichtleiter nicht nur an den Enden sondern auch über den Längsverlauf des Schlauches fixiert werden können. Dies kann über Teilbereiche, mehrere Teilbereiche oder dergleichen erfolgen, um die zuvor erwähnten Temperaturspannungsdehnungen nach wie vor zu erlauben.

In einer weiteren Ausgestaltung der Erfindung werden die Verklebungen derart bewerkstelligt, dass die Lichtleiter abdichtend im Bereich der Schlauchenden fixiert werden.

Diese Maßnahme hat den Vorteil, dass diese Fixierstellen gleichzeitig als Abdichtstellen dienen, so dass ausgeschlossen ist, dass irgendwelche Kontaminationen, insbesondere Dampf oder dergleichen, in das Innere des Schlauches dringen können.

In einer weiteren Ausgestaltung der Erfindung werden bei einem hohlzylindrischen zweiten Kanal mehrere, die Lichtleiterfasern umhüllende Schläuche umfänglich verteilt eingebracht.

Diese Maßnahme hat den Vorteil, dass bei dieser räumlichen Konstellation mehrere Stränge an Schläuchen mit darin aufgenommenen lichtleitenden Fasern eingesetzt werden können.

Dies ist besonders vorteilhaft zu erzielen, wenn etwa vier um jeweils 90° umfänglich zueinander versetzte Schläuche eingebracht werden.

In einer weiteren Ausgestaltung der Erfindung wird bei einem hohlzylindrischen zweiten Kanal ein Zusammenbau aus Schlauch und Lichtleitern bewerkstelligt, der einen mittigen Kern aufweist.

Diese Maßnahme hat den Vorteil, dass eine Möglichkeit geschaffen wird, den gesamten hohlzylindrischen zweiten Kanal gleichmäßig mit Lichtleitern zu füllen. Der mittige Kern wird dabei so gewählt, dass er in den Ausmaßen dem späteren Innenrohr des Endoskopes entspricht. Es ist dadurch möglich, außerhalb des Endoskopes die einzelnen Lichtleitfasern in entsprechend gleichmäßig verteilter Form in dem Hohlraum zwischen dem mittigen Kern und dem äußeren Schlauch zu verteilen und gegebenenfalls auch zu fixieren, und dann diesen Zusammenbau von dem Kern direkt in das Endoskop einzuschieben. Dabei kann der Kern an das Innenrohr angesetzt werden, und der Zusammenbau vom Kern abgezogen und gleich auf das Innenrohr aufgeschoben werden.

In einer vorteilhaften Ausgestaltung ist vorgesehen, die Lichtleiter mit einem Klebemittel zu benetzen und das Klebemittel mit noch eingeschobenem Kern auszuhärten.

Diese Maßnahme hat den Vorteil, dass durch das Klebemittel die Lichtleiter in der geometrischen Anordnung des hohlzylindrischen Körpers fixiert werden. Durch diese Vorhärtung und dem Zustand, dass gegebenenfalls Lichtleitfasern auf ihrer gesamten Länge fest mit dem Schlauch verbunden bzw. verklebt sind, kann bei Wärmedehnung, die sich bei den Lichtleitfasern und dem Endoskopschaft deutlich unterscheidet, der Schlauch dadurch, dass Lichtleitfasern unter Spannung gesetzt sind, eventuell Schäden bzw. Risse erleiden. Um eine dadurch mögliche Undichtigkeit von außen zum Faserbündel hin zu verhindern, können um den das Faserbündel direkt umgebenden Schlauch gegebenenfalls weitere Schläuche um diesen herum vorgesehen werden.

In einer weiteren Ausgestaltung ist über dem Kern ebenfalls ein Schlauch angebracht.

Diese Maßnahme hat den Vorteil, dass ein hohlzylindrisches Gebilde vorgeformt wird, das sowohl an der inneren als auch der äußeren zylindrischen Wand einen solchen Schlauch aufweist, zwischen denen dann die Lichtleitfasern aufgenommen sind.

In einer weiteren Ausgestaltung der Erfindung wird der Schlauch zunächst als rechteckförmiger Streifen bereitgestellt, auf den die Lichtleiter aufgelegt werden, und der Streifen wird anschließend um die Lichtleiter herum zu einem Schlauch geschlossen.

Diese Maßnahme hat den Vorteil, dass bei extremen geometrischen Ausgestaltungen, beispielsweise bei extrem langen und dünnen Endoskopen, nur ein äußerst geringer lichter Durchmesser des zweiten Kanals zur Verfügung steht, und dass es dann Mühe bereiten könnte, die Lichtleiterbündel in einen solchen dünnen und gegebenenfalls langen Kanal einzubringen. Der so geformte Zusammenbau wird dann wie zuvor beschrieben weiter gehandhabt.

In einer weiteren Ausgestaltung der Erfindung wird während der Montage in ein Schlauchende eine trichterförmig aufgeweitete Einschubhilfe eingesteckt, über diese Einschubhilfe werden die Lichtleiter in den Schlauch eingeschoben, und anschließend wird die Einschubhilfe abgenommen.

Diese Maßnahme hat den Vorteil, dass das Einführen des Lichtleiterbündels in den Schlauch sehr vereinfacht ist. Dabei kann der Schlauch hängend angeordnet werden, an dessen oberem Ende die Einschubhilfe eingesteckt und dann die Lichtleitfaser von oben sanft eingeschoben werden.

Es ist auch möglich, den Schlauch vor Einbringen in den zweiten Kanal vorzuschrumpfen und nach dem Einbringen erneut zu schrumpfen.

In einer weiteren Ausgestaltung der Erfindung wird das Härten des Klebemittels und das Schrumpfen des Schlauchs gleichzeitig unter Einwirkung von Wärme durchgeführt.

Diese Maßnahme hat den Vorteil, dass eine einfache und Energie sparende Herstellung möglich ist, bei der sowohl der Schlauch geschrumpft wird als auch das Klebemittel ausgehärtet wird, so dass durch einen einzigen Wärmebehandlungsvorgang ein entsprechend vorgefertigter Zusammenbau bewerkstelligt werden kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend an Hand einiger ausgewählter Ausführungsbeispiele im Zusammenhang mit den beiliegenden Zeichnungen näher beschrieben und erläutert. Es zeigen:
- Fig. 1: stark schematisch einen Längsschnitt eines Endoskopes während einer Montage, bei dem bereits ein Zusammenbau aus flexiblem Schlauch und Lichtleitern in den zweiten Kanal eingesetzt ist,
- Fig. 2: einen Schnitt längs der Linie II-II in Fig. 1,
- Fig. 3: einen Vormontagezustand, und zwar den Zusammenbau aus flexiblem Schlauch und Lichtleiter, der in Fig. 1 in das Endoskop eingesetzt ist,
- Fig. 4: eine der Darstellung von Fig. 3 vergleichbare Darstellung eines Vormontage-Zusammenbaus aus flexiblem Schlauch und Lichtleitern für ein Endoskop mit einem hohlzylinderförmigen zweiten Kanal,
- Fig. 5: einen dem von Fig. 2 vergleichbaren Schnitt eines Endoskopschaftes, in dem ein Zusammenbau, wie er in Fig. 4 dargestellt ist, eingesetzt wurde,
- Fig. 6: einen der Darstellung von Fig. 5 vergleichbaren Schnitt eines Endoskopes mit hohlzylinderförmigem zweiten Kanal, in dem vier umfänglich verteilte flexible Schläuche mit darin enthaltenen Lichtleitern eingesetzt sind,
- Fig. 7: eine Draufsicht auf eine Vormontagezustand eines Zusammenbaus aus einem rechteckigen Streifen und Lichtleitern, wobei der rechteckige Streifen zu einem Schlauch gerollt werden soll, und
- Fig. 8: eine der Fig. 7 vergleichbare Darstellung, nachdem der rechteckige Streifen zu einem Schlauch geformt worden ist.

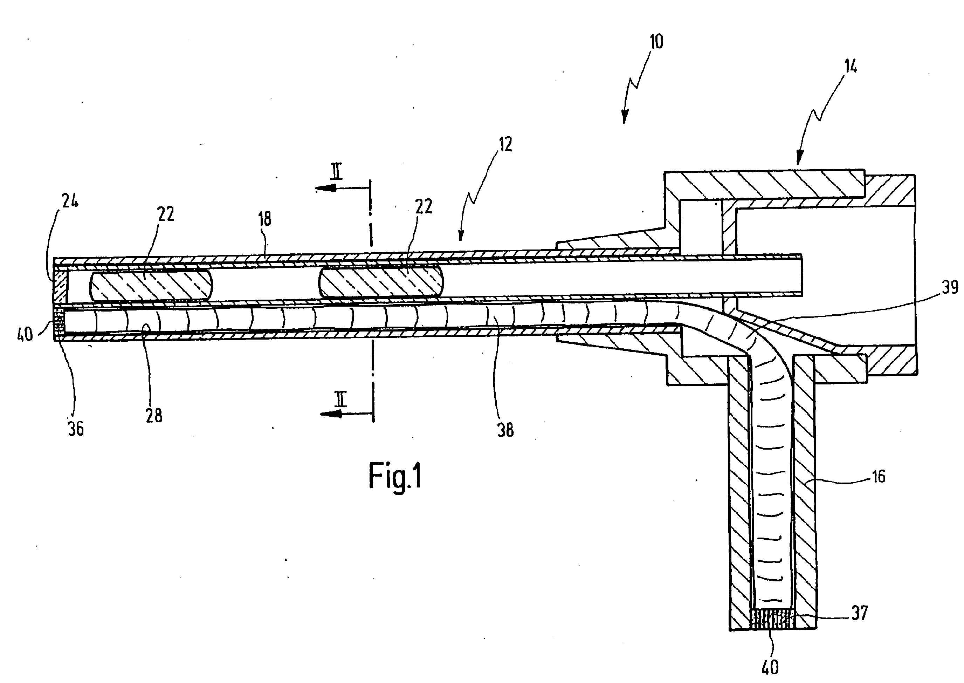

Ein in Fig. 1 dargestelltes Endoskop ist in seiner Gesamtheit mit der Bezugsziffer 10 versehen.

Das Endoskop 10 weist einen langerstreckten Schaft 12 auf, der proximalseitig mit einem Gehäuse 14 versehen ist.

Seitlich etwa um 90° zur Längsachse des Schaftes 12 abgewinkelt erstreckt sich ein Lichtleiteranschluss 16 vom Gehäuse 14 weg. Das Endoskop 10 ist im Bereich seines Gehäuses 14 noch nicht im endmontierten Zustand dargestellt, der Übersicht halber sind die im Gehäuse 14 enthaltenen optischen Elemente und die abschließende Okularmuschel nicht dargestellt.

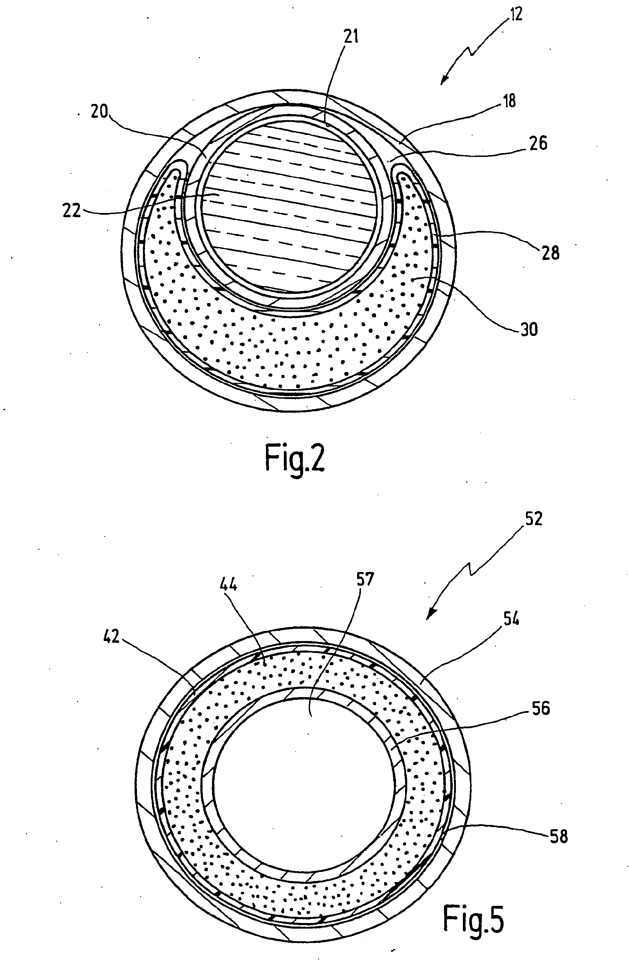

Der Schaft 12 des Endoskopes 10, wie das auch aus der Schnittdarstellung von Fig. 2 ersichtlich ist, besteht aus einem Außenrohr 18, in dem ein durchmessergeringeres Innenrohr 20 aufgenommen ist. Das Innenrohr 20 ist außermittig angeordnet, d.h. es liegt längs einer äußeren Mantellinie an einer Innenseite des Außenrohres 18 an, wie das insbesondere aus der Schnittdarstellung von Fig. 2 ersichtlich ist.

Der Innenraum des Innenrohres 20 stellt einen ersten rohrförmigen Kanal 21 zur Aufnahme der bildleitenden Bauelemente des Endoskopes 10 dar.

In Fig. 1 sind lediglich beispielhaft einige dieser bildleitenden Elemente dargestellt, nämlich zwei Stablinsen 22 und ein den ersten rohrförmigen Kanal 21 proximal abschließendes Abschlussfenster 24. Zwischen der Außenseite des Innenrohrs 20 und der Innenseite des Außenrohrs 18 ist ein zweiter Kanal 26 ausgebildet, der im Bereich des Schaftes 12 einen mondsichelförmigen Querschnitt aufweist, wie das insbesondere aus Fig. 2 ersichtlich ist.

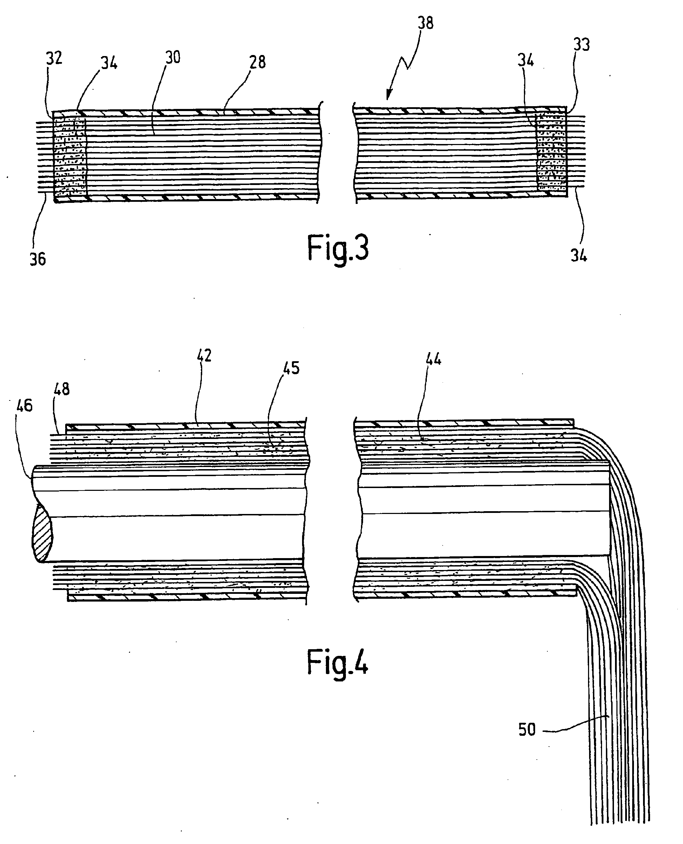

In dem sichelförmigen zweiten Kanal 26 ist ein Zusammenbau 38 aus einem flexiblen Schlauch 28 und darin enthaltenen Lichtleitern 30 in Form eines Bündels aus Glasfasern aufgenommen. Der Aufbau des Zusammenbaus 38 soll zunächst an Hand von Fig. 3 näher beschrieben werden.

Der Zusammenbau 38 besteht, wie erwähnt, aus einem flexiblen Schlauch 28 aus Kunststoffmaterial. Im dargestellten Ausführungsbeispiel ist das Material ein schrumpffähiges Kunststoffmaterial.

In den flexiblen Schlauch 28 ist ein Bündel an Glasfasern eingeführt, dessen Länge so gewählt ist, dass die gegenüberliegenden Enden 32 und 33 des flexiblen Schlauches 28 geringfügig, etwa um 1 cm, überragt werden. Das Bündel aus Glasfasern stellt den Lichtleiter 30 dar. Im Bereich der Enden 32 und 33 sind die Glasfasern über ein Klebemittel 34 untereinander und mit der Innenseite des flexiblen Schlauches 28 verbunden. Dabei wird das Klebemittel 34 so aufgetragen und so gewählt, dass ein dichtender Abschluss gewährt ist, so dass von der Außenseite keine Kontaminationen wie Feuchtigkeit oder auch überhitzter Wasserdampf in das Innere des flexiblen Schlauchs 28 eintreten können.

Zum Einbringen der Lichtleiter 30 in den flexiblen Schlauch 28 kann dieser lotrecht hängend ausgerichtet werden, und in ein Ende, nämlich in das obere Ende, beispielsweise das Ende 33, eine trichterförmig nach außen aufgeweitete Einschubhilfe eingesteckt werden. Über die Einschubhilfe werden die Lichtleiter 30 als Glasfaserbündel von oben nach unten eingeführt. Dazu werden die Glasfasern mit einem leicht verdampfbaren Anfeuchtungsmittel etwas angefeuchtet, wodurch diese zu einem nicht aufgespleißten, etwa stabförmigen Bündel durch Adhäsionskräfte aneinander gehalten werden. Nach Einbringen der Lichtleiter 30 wird jeweils im Bereich der Enden 32 und 33 das Klebemittel 34 eingebracht und gegebenenfalls unter Zuhilfenahme von Wärme ausgehärtet. Ein mögliches Schrumpfen des flexiblen Schlauches 28 aus schrumpffähigem Material bei dieser Aushärtung im Bereich der Enden 32 und 33 wird dabei in Kauf genommen.

Der in Fig. 3 dargestellte Zusammenbau 38 wird nunmehr in den zweiten Kanal 26 des Endoskopes 10 eingebracht z.B. eingeschoben, und zwar von distal nach proximal, also in der Darstellung von Fig. 1 von links nach rechts. Der Zusammenbau 38 kann dabei in völlig gerader Ausrichtung eingeschoben oder auch eingedreht werden, so dass er am proximalen Ende des noch offenen Gehäuses 14 herausragt. In diesem Montagezustand ist der seitliche Lichtleiteranschluss 16 noch nicht in das Gehäuse 14 eingesetzt. Durch sanftes Biegen des Zusammenbaus 38 wird dieser seitlich abgebogen und aus der entsprechenden Öffnung, in die der Lichtleiteranschluss 16 eingedreht wird, hinausgeschoben. Dann wird der Lichtleiteranschluss 16 über dieses seitlich abhängende Ende des Zusammenbaus 38 aufgeschoben und eingesetzt. Der Umfang des flexiblen Schlauches 28 ist nunmehr so gewählt, dass er sich der sichelförmigen Querschnittskontur des zweiten Kanals 28 anschmiegen kann, wie das insbesondere aus der Schnittdarstellung von Fig. 2 hervorgeht. Dadurch sind dann die einzelnen Glasfasern des Bündels, die insgesamt den Lichtleiter 30 aufbauen, gleichmäßig in dem sichelförmigen Bereich verteilt.

Über den seitlichen Lichtleiteranschluss 16 wird der Schlauch 28 mit kreisförmigem Querschnitt abgeführt.

Aus Fig. 1 ist zu entnehmen, dass in dem gekrümmten Übergangsbereich 39 die Lichtleiter 30 durch den flexiblen Schlauch 28 geschützt sind. Es kann nun in diesem Montagezustand das Schrumpfen des Schlauches bewirkt werden, es kann auch zunächst so verfahren werden, dass die überstehenden Enden 36 und 37 mit Klebemitteln 40 versetzt werden und zu einem dichten Abschlussstopfen verklebt werden, der am distalen Ende dann die Querschnittsform einer mondförmigen Sichel in den Maßen des zweiten Kanals 26 und am proximalen, seitlich abgebogenen Ende die Querschnittsform des lichten Innenraums des Lichtleiteranschlusses 16 aufweist. Es kann aber auch erst zu diesem Zeitpunkt der Schlauch 28 geschrumpft werden, wobei dies durch eine gemeinsame Wärmebehandlung durchgeführt werden kann, die zum Aushärten des Klebemittels 40 und zugleich zum Schrumpfen dient.

Die Lichtleiter 30 im Inneren des Schlauches 28 sind rundum geschützt und lediglich über die endseitigen proximalen und distalen Klebepunkte befestigt. Dadurch sind gewisse Bewegungen, insbesondere Wärmedehnungen bei den Sterilisierzyklen, möglich, falls das Endoskop 10 für den medizinischen Bereich eingesetzt wird. Wird es im industriellen Bereich eingesetzt, kann es ebenfalls die entsprechenden Wärmedehnungsspannungen ausgleichen, beispielsweise wenn es in einem warmen oder heißen Raum eingesetzt wird.

Das Material und die Geometrie des flexiblen Schlauches 28 können so ausgewählt sein, dass durch den Schrumpfvorgang verschiedene Fixierstellen mit der Innenwand des zweiten Kanals 26 bzw. des Lichtleiteranschlusses 16 ausgebildet werden. Dazu können beispielsweise im Material des Schlauches 28 Wülste oder Wölbungen vorgesehen sein, die beim radialen Zusammenziehen beim Schrumpfen ein gewisses axiales Verklemmen mit den den flexiblen Schlauch 28 umgebenden Wänden besorgen.

Bei dem in Fig. 4 dargestellten Ausführungsbeispiel ist ein flexibler Schlauch 42 vorgesehen, in dessen Innenraum Lichtleiter 44 eingebracht sind, wobei auch hier die Lichtleiter 44 aus einem Bündel an feinen lichtleitenden Glasfasern bestehen. Die Lichtleiter 44 wurden zuvor über ihre gesamte Länge mit einem Klebemittel 45 getränkt. Mittig wird ein Kern 46 eingetrieben, wobei zum Erleichtern des Einschiebens dieser gegebenenfalls eine Spitze aufweisen kann. Die Länge der Lichtleiter 44 ist dabei so gewählt, dass diese an einem Ende, das später das distalseitige Ende ist, etwas überstehen, etwa 1 cm, und am gegenüberliegenden Ende ein längerer freiliegender Strang 50 vorhanden ist. Nach Aushärten der Klebemittel 45 entsteht um den Kern 46 herum ein Lichtleitergebilde in Form eines Hohlzylinders, wie er in dem Querschnitt von Fig. 5 ersichtlich ist.

Der dort dargestellte Schaft 52 eines Endoskopes weist ein Außenrohr 54 auf, in dem konzentrisch ein Innenrohr 56 aufgenommen ist. Der Innenraum des Innenrohrs 56 umgrenzt hier wiederum einen ersten Kanal 57, der zur Aufnahme des bildleitenden Systems, also beispielsweise der zuvor beschriebenen Linsen etc., vorgesehen ist.

Der im Querschnitt ringförmige Raum um die Außenseite des Innenrohrs 56 und die Innenseite des Außenrohrs 54 stellt den zweiten Kanal 58 zur Aufnahme des lichtleitenden Systems dar.

Daher weist der Kern 46 einen Außendurchmesser auf, der in etwa dem Außendurchmesser des Innenrohrs 56 entspricht. Bei der Montage kann nun der in Fig. 4 dargestellte Zusammenbau beispielsweise von proximal durch das Gehäuse des Endoskopes hindurchgeschoben und so angelegt werden, dass der Kern 46 in etwa in Ausrichtung mit dem Innenrohr 46 steht. Dann kann der Zusammenbau aus Lichtleiter 44 und flexiblem Schlauch 42 direkt vom Kern 46 abgeschoben und in den zweiten Kanal 58 eingeschoben werden. Der freiliegende Strang 50 kann dann in den seitlichen Lichtleiteranschluss eingeschoben werden; es kann auch zuvor noch ein entsprechender Schlauch übergeschoben werden. Es ist auch möglich, über die Außenseite des Schaftes 52 einen weiteren Schlauch zu schieben, so dass dann der hohlzylindrische Zusammenbau sowohl auf der Innenseite als auch auf der Außenseite einen entsprechenden flexiblen Schlauch aufweist. Auch hier dienen die distal überstehenden Enden 48 der Glasfasern dazu, um mit dem distalen Ende des Endoskopes abschließend dichtend verklebt zu werden, wie das zuvor in der Ausführung von Fig. 1 beschrieben worden ist. Auch hier ist vorgesehen, den flexiblen Schlauch 42 aus schrumpffähigem Material herzustellen, und den Zusammenbau vor Einschieben in das Endoskop zu einem kompakten Zusammenbau zu schrumpfen.

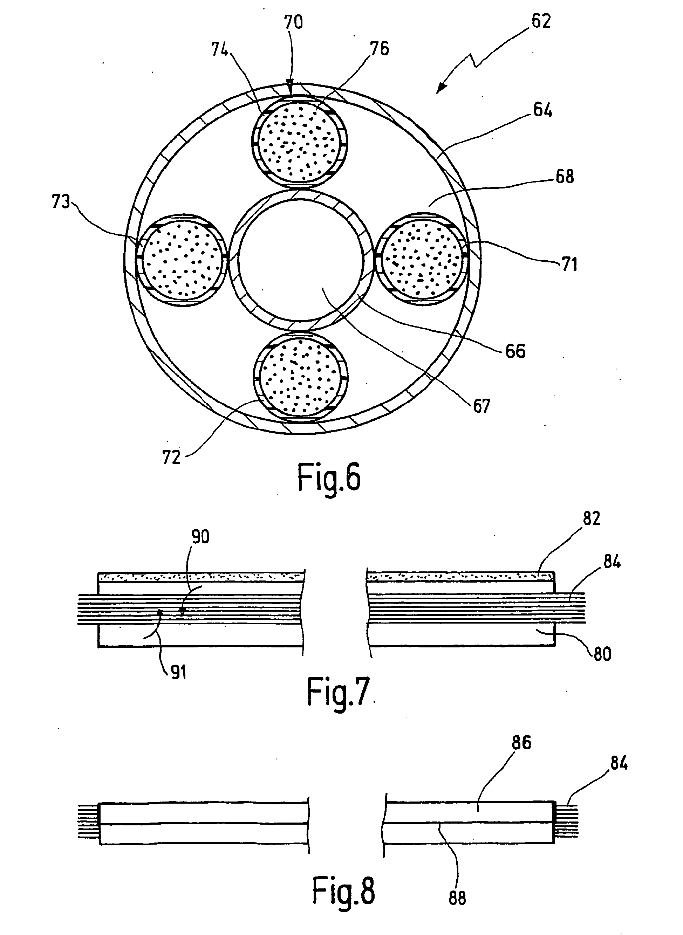

In Fig. 6 ist ein Querschnitt eines Schaftes 62 dargestellt, der ähnlich wie zuvor beschrieben aus einem Außenrohr 64 und einem konzentrisch dazu angeordneten Innenrohr 66 ausgebildet ist. Das Innenrohr 66 umgrenzt wieder den ersten Kanal 67 zur Aufnahme des bildleitenden Systems. Auch hier existiert dann wieder ein zweiter Kanal 68, der von der Außenseite des Innenrohrs 66 und der Innenseite des Außenrohrs 64 umgrenzt ist und von hohlzylindrischer Form ist. In Fig. 6 ist ersichtlich, dass in diesen hohlzylindrischen zweiten Kanal 68 nun vier Zusammenbauexemplare 70, 71, 72, 73 eingeschoben sind, wie sie zuvor in Fig. 3 beschrieben worden sind. Diese sind dabei so angeordnet, dass diese jeweils um 90° zueinander versetzt sind. Jeder einzelne Zusammenbau, wobei hier stellvertretend nur der Zusammenbau 70 bezeichnet ist, besteht dann wieder aus einem flexiblen Schlauch 74, in dessen Innerem ein Lichtleiter 76 aus einem Bündel an feinen Glasfasern zusammengesetzt ist. Die Halterung kann auf mechanischem Wege bestehen, nämlich durch die Distanzhalter, die das Innenrohr 66 in der koaxialen Ausrichtung zum Außenrohr 64 halten. Diese Distanzhalter bestehen aus zumindest zwei oder auch mehr axial voneinander beabstandeten Ringen, die auf das Innenrohr 66 aufgeschoben sind und die vier um 90° versetzte Durchgangsöffnungen aufweisen, durch die die Zusammenbauexemplare 70, 71, 72, 73 hindurchgeschoben sind.

Aus der Schnittdarstellung von Fig. 6 ist ersichtlich, dass zwischen den einzelnen Zusammenbauexemplaren weitere kanalartige Öffnungen frei sind, die zur Aufnahme von weiteren Kanälen oder Leitungen herangezogen werden, beispielsweise, um Spülkanäle für Spülflüssigkeiten oder Saugkanäle zum Absaugen von Flüssigkeiten oder Gasen heranziehen zu können.

In den Fig. 7 und 8 ist dargestellt, wie ein zuvor beschriebener Zusammenbau aus Schlauch und Lichtleiter bewerkstelligt werden kann, wenn ein extrem langes und dünnes Endoskop vorliegt. Hier kann es gegebenenfalls schwierig sein, die Lichtleiter in einen Innenraum eines Schlauches sicher einzuführen.

Dazu ist ein entsprechender rechteckförmiger Streifen 80 vorgesehen, der über eine Längskante mit einem Kleberand 82 versehen ist. Auf den Streifen 80 wird der Lichtleiter 44, also ein Bündel an Glasfasern, aufgelegt, und anschließend wird der Streifen 80 zu einem Schlauch geformt, wobei zunächst das eine Ende umgeschlagen wird, wie das durch einen Pfeil 89 dargestellt ist, und anschließend das andere Ende dieses überlappend umgeschlagen wird, wie das durch einen Pfeil 90 dargestellt ist, wodurch dann durch den Kleberand 82 eine Fixierung zu einem schlauchförmigen Körper bewerkstelligt wird.

Dieser Zusammenbau weist zwar dann eine Nahtstelle 88 auf, was bei einem entsprechend dünnen Schlauchmaterial aber nicht störend ist.

## Patentansprüche

1. Verfahren zum Montieren eines Endoskopes (10), mit einem ersten rohrförmigen Kanal (21, 57, 67) zur Aufnahme von bildleitenden Bauelementen (22), und mit einem zweiten Kanal (26, 58, 68) zur Aufnahme von Lichtleitern (30, 44, 76, 84), wobei die Lichtleiter (30, 44, 76, 84) in einen flexiblen Schlauch (28, 42, 74, 86) eingebracht werden und der Zusammenbau (38, 70-73) aus Schlauch (28, 42, 74, 86) und Lichtleiter (30, 44, 76, 84) in den zweiten Kanal (26, 58, 68) eingebracht werden, **dadurch gekennzeichnet, dass** die Lichtleiter (30, 44, 76, 84) im Schlauch (42) fixiert werden, und die Lichtleiter (30, 44, 76, 84) mit einer solchen Länge versehen werden, dass sie beidseits des Schlauches (28, 42, 74, 86) hervorragen, und wobei ein Schlauch (28, 42, 74, 86) eingesetzt wird, der aus schrumpffähigen Material besteht und der Schlauch (28) nach dem Einbringen in den zweiten Kanal geschrumpft wird.

2. Verfahren nach Anspruch 1, **dadurch gekennzeichnet, dass** die Lichtleiter (30) im Bereich der Schlauchenden (32, 33) fixiert werden.

3. Verfahren nach Anspruch 1 oder 2, **dadurch gekennzeichnet, dass** die Lichtleiter (44) über ein Klebemittel (45) im Schlauch (42) fixiert werden.

4. Verfahren nach Anspruch 3, **dadurch gekennzeichnet, dass** die Verklebungen derart bewerkstelligt werden, dass die Lichtleiter (30) abdichtend im Bereich der Schlauchenden (32, 33) fixiert werden.

5. Verfahren nach einem der Ansprüche 1 bis 4, **dadurch gekennzeichnet, dass** bei einem hohlzylindrischen zweiten Kanal (68) mehrere Schläuche (74) umfänglich verteilt eingebracht werden.

6. Verfahren nach Anspruch 5, **dadurch gekennzeichnet, dass** vier Schläuche (74) jeweils um ca. 90° umfangsversetzt eingebracht werden.

7. Verfahren nach einem der Ansprüche 1 bis 4, **dadurch gekennzeichnet, dass** bei einem hohlzylindrischen zweiten Kanal (58) ein Zusammenbau aus Schlauch (42) und Lichtleiter (44) bewerkstelligt wird, der einen mittigen Kern (46) aufweist.

8. Verfahren nach Anspruch 7, **dadurch gekennzeichnet, dass** die Lichtleiter (44) mit einem Klebemittel (45) benetzt sind und das Klebemittel (45) noch mit eingeschobenem Kern (46) ausgehärtet wird.

9. Verfahren nach Anspruch 7 oder 8, **dadurch gekennzeichnet, dass** über den Kern (46) ebenfalls ein Schlauch gebracht wird.

10. Verfahren nach Anspruch 1, **dadurch gekennzeichnet, dass** der Schlauch vor dem Einbringen in den zweiten Kanal vorgeschrumpft ist.

11. Verfahren nach einem der Ansprüche 3 bis 10, **dadurch gekennzeichnet, dass** das Härten des Klebemittels und das Schrumpfen des Schlauches gleichzeitig unter Einwirkung von Wärme durchgeführt wird.

12. Endoskop mit einem ersten rohrförmigen Kanal (21, 57, 67) zur Aufnahme von bildleitenden Bauelementen (22), und mit einem zweiten Kanal (26, 58, 68) zur Aufnahme von Lichtleitern (30, 44, 76, 84), und der Zusammenbau (38, 70-73) aus Lichtleiter (30, 44, 76, 84) und Schlauch (28, 42, 74, 86) im zweiten Kanal (26, 58, 68) aufgenommen ist, **dadurch gekennzeichet, dass** die Lichtleiter (30, 44, 76, 84) in einem geschrumpften flexiblen Schlauch (28, 42, 74, 86) aufgenommen sind, und wobei die Lichtleiter (30, 44, 76, 84) im Schlauch fixiert sind und eine solche Länge aufweisen, dass sie beidseits des Schlauches hervorragen.

13. Endoskop nach Anspruch 12, **dadurch gekennzeichnet, dass** die Lichtleiter (30) im Bereich der Schlauchenden (32, 33) fixiert sind.

14. Endoskop nach Anspruch 12 oder 13, **dadurch gekennzeichnet, dass** die Lichtleiter (44) über ein Klebemittel (45) im Schlauch (42) fixiert sind.

15. Endoskop nach Anspruch 14, **dadurch gekennzeichnet, dass** die Verklebungen derart sind, dass die Lichtleiter (30) abdichtend im Bereich der Schlauchenden (32, 33) fixiert sind.

16. Endoskop nach einem der Ansprüche 12 bis 15, **dadurch gekennzeichnet, dass** der flexible Schlauch (28) nach dem Schrumpfvorgang im zweiten Kanal (26) fixiert ist.

## Claims

1. Method for assembling an endoscope (10), with a first tubular channel (21, 57, 67) for receiving image-transmitting components (22), and with a second channel (26, 58, 68) for receiving light guides (30, 44, 76, 84), wherein the light guides (30, 44, 76, 84) are introduced into a flexible sleeve (28, 42, 74, 86), and the unit (38, 70-73) made up of sleeve (28, 42, 74, 86) and light guides (30, 44, 76, 84) is introduced into the second channel (26, 28, 68), **characterized in that** the light guides (30, 44, 76, 84) are fixed in the sleeve (42), and **in that** the light guides (30, 44, 76, 84) are provided with such a length that they protrude from both ends of the sleeve (28, 42, 74, 86), and **in that** a sleeve (28, 42, 74, 86) is used which is made of a shrinkable material, and the sleeve (28) is shrunk after introduction into the second channel.

2. Method of claim 1, **characterized in that** the light guides (30) are fixed in the area of the sleeve ends (32, 33).

3. Method of claims 1 or 2, **characterized in that** the light guides (44) are fixed in the sleeve (42) by an adhesive (45).

4. Method of claim 3, **characterized in that** the adhesive connections are made in such a way that the light guides (30) are fixed sealingly in the area of the sleeve ends (32, 33).

5. Method of anyone of claims 1 through 4, **characterized in that** in case of a hollow cylindrical second channel (68), a plurality of sleeves (74) are distributed around the circumference.

6. Method of claim 5, **characterized in that** four sleeves (74) each offset by about 90° are arranged around the circumference.

7. Method of anyone of claims 1 through 4, **characterized in that** in case of a hollow cylindrical second channel (28), a unit made up of sleeve (42) and light guides (44) is produced which has a central core (46).

8. Method of claim 7, **characterized in that** the light guides are wetted with an adhesive (45), and the adhesive (45) is cured with the core (46) still inserted.

9. Method of claims 7 or 8, **characterized in that** a sleeve is also fitted over the core (46).

10. Method of claim 1, **characterized in that** the sleeve is pre-shrunk before introduction into the second channel.

11. Method of anyone of claims 3 through 10, **characterized in that** the curing of the adhesive and the shrinking of the sleeve are carried out simultaneously under the effect of heat.

12. Endoscope, with a first tubular channel (21, 57, 67) for receiving image transmitting components (22), and with a second channel (26, 58, 68) for receiving light guides (30, 44, 76, 84), and the unit (38, 70-73) made up of light guides (30, 44, 76, 84) and sleeve (28, 42, 74, 86) is received in the second channel (26, 58, 68), **characterized in that** the light guides (30, 44, 76, 84) are received in a shrunk flexible sleeve (28, 42, 74, 86), and **in that** the light guides (30, 44, 76, 84) are fixed within the sleeve and are provided with such a length that they protrude from both ends of the sleeve.

13. Endoscope of claim 12, **characterized in that** the light guides (30) are fixed in the area of the sleeve ends (32, 33).

14. Endoscope of claim 12 or 13, **characterized in that** the light guides (44) are fixed in the sleeve (42) by an adhesive (45).

15. Endoscope of claim 14, **characterized in that** the adhesive connections are made in such a way that the light guides (30) are fixed sealingly in the area of the sleeve ends (32, 33).

16. Endoscope of anyone of claims 12 through 15, **characterized in that** the flexible sleeve (28) is fixed in the second channel (26) after the shrinking process.

## Revendications

1. Procédé pour assembler un endoscope (10) comprenant un premier canal tubulaire (21, 57, 67) recevant des composants conducteurs d'images (22) et un deuxième canal (26, 58, 68) recevant des conducteurs de lumière (30, 44, 76, 84), les conducteurs de lumière (30, 44, 76, 84) étant introduits dans un tuyau flexible (28, 42, 74, 86) et l'ensemble (38, 70-73) composé du tuyau (28, 42, 74, 86) et des conducteurs de lumière (30, 44, 76, 84) étant introduit dans le deuxième canal (26, 58, 68), **caractérisé en ce que** les conducteurs de lumière (30, 44, 76, 84) sont fixés dans le tuyau (42) et les conducteurs de lumière (30, 44, 76, 84) sont pourvus d'une longueur telle qu'ils dépassent des deux côtés du tuyau (28, 42, 74, 86), un tuyau (28, 42, 74, 86) en matériau rétractable étant utilisé et le tuyau (28) étant rétracté après l'introduction dans le deuxième canal.

2. Procédé selon la revendication 1, **caractérisé en ce que** les conducteurs de lumière (30) sont fixés dans la région des extrémités de tuyau (32, 33).

3. Procédé selon la revendication 1 ou 2, **caractérisé en ce que** les conducteurs de lumière (44) sont fixés dans le tuyau (42) au moyen d'un produit adhésif (45).

4. Procédé selon la revendication 3, **caractérisé en ce que** les collages sont réalisés de manière que les conducteurs de lumière (30) soient fixés de manière étanche dans la région des extrémités de tuyau (32, 33).

5. Procédé selon l'une des revendications 1 à 4, **caractérisé en ce que** dans le cas d'un deuxième canal cylindrique creux (68), plusieurs tuyaux (74) sont introduits en étant répartis sur la circonférence.

6. Procédé selon la revendication 5, **caractérisé en ce que** quatre tuyaux (74) décalés chaque fois d'environ 90° sur la circonférence sont introduits.

7. Procédé selon l'une des revendications 1 à 4, **caractérisé en ce que** dans le cas d'un deuxième canal cylindrique creux (58), un ensemble composé du tuyau (42) et de conducteurs de lumière (44), qui présente un noyau central (46), est réalisé.

8. Procédé selon la revendication 7, **caractérisé en ce que** les conducteurs de lumière (44) sont imprégnés d'un produit adhésif (45) et le produit adhésif (45) est durci avec le noyau (46) encore inséré.

9. Procédé selon la revendication 7 ou 8, **caractérisé en ce qu'**un tuyau est également monté au-dessus du noyau (46).

10. Procédé selon la revendication 1, **caractérisé en ce que** le tuyau est pré-rétracté avant l'introduction dans le deuxième canal.

11. Procédé selon l'une des revendications 3 à 10, **caractérisé en ce que** le durcissement du produit adhésif et la rétraction du tuyau sont effectués en même temps sous l'action de la chaleur.

12. Endoscope comprenant un premier canal tubulaire (21, 57, 67) recevant des composants conducteurs d'images (22) et un deuxième canal (26, 58, 68) recevant des conducteurs de lumière (30, 44, 76, 84), l'ensemble (38, 70-73) composé des conducteurs de lumière (30, 44, 76, 84) et d'un tuyau (28, 42, 74, 86) étant logé dans le deuxième canal (26, 58, 68), **caractérisé en ce que** les conducteurs de lumière (30, 44, 76, 84) sont logés dans un tuyau flexible rétracté (28, 42, 74, 86), les conducteurs de lumière (30, 44, 76, 84) étant fixés dans le tuyau et présentant une longueur telle qu'ils dépassent des deux côtés du tuyau.

13. Endoscope selon la revendication 12, **caractérisé en ce que** les conducteurs de lumière (30) sont fixés dans la région des extrémités de tuyau (32, 33).

14. Endoscope selon la revendication 12 ou 13, **caractérisé en ce que** les conducteurs de lumière (44) sont fixés dans le tuyau (42) au moyen d'un produit adhésif (45).

15. Endoscope selon la revendication 14, **caractérisé en ce que** les collages sont réalisés de manière que les conducteurs de lumière (30) soient fixés de manière étanche dans la région des extrémités de tuyau (32, 33).

16. Endoscope selon l'une des revendications 12 à 15, **caractérisé en ce que** le tuyau flexible (28) est fixé dans le deuxième canal (26) après l'opération de rétraction.
